# FASCICULE DE BREVET EUROPEEN

(11) **EP 2 016 422 B1**
(45) Date de publication et mention de la délivrance du brevet: **03.09.2014**
(21) Numéro de dépôt: 07731410.2
(22) Date de dépôt: 03.05.2007
(51) Int. Cl.: G01N 33/86, C12Q 1/56

(54) **DETECTION DES MALADIES VEINEUSES THROMBOEMBOLIQUES PAR LE DOSAGE DES D-DIMERES ET DE LA FIBRINE SOLUBLE**
NACHWEIS VENÖSER THROMBOEMBOLISCHER KRANKHEITEN DURCH MESSUNG VON D-DIMEREN UND LÖSLICHEN FIBRINNIVEAUS
DETECTION OF VENOUS THROMBOEMBOLIC DISEASES BY MEASUREMENT OF D-DIMERS AND SOLUBLE FIBRIN LEVELS

(30) Priorité: 05.05.2006 FR 0604072
(43) Date de publication de la demande: 21.01.2009
(73) Titulaire: Diagnostica Stago, 92600 Asnières (FR); Assistance Publique - Hôpitaux de Paris, 75100 Paris (FR)
(72) Inventeur: MIRSHAHI, Bibi, Shah, Soltan, 95210 Saint Gratien (FR); SORIA, Jeannette, 95150 Taverny (FR)
(74) Mandataire: Desaix, Anne
(86) Numéro de dépôt international: PCT/FR2007/000764
(87) Numéro de publication internationale: WO 2007/128916

(56) Documents cités:
- EP-A2- 0 347 933
- WO-A-02/18628
- US-A- 5 114 845
- BRIMBLE K SCOTT ET AL: "Evaluation of the combination of a bedside D-dimer assay and enzyme-linked immunosorbent soluble fibrin assay in patients with suspected venous thromboembolism" THROMBOSIS RESEARCH, vol. 88, no. 3, 1 novembre 1997 (1997-11-01), pages 291-297, XP002408680 ISSN: 0049-3848
- OTA SATOSHI ET AL: "Diagnosis of deep vein thrombosis by plasma-soluble fibrin or D-dimer" AMERICAN JOURNAL OF HEMATOLOGY, vol. 79, no. 4, août 2005 (2005-08), pages 274-280, XP002408681 ISSN: 0361-8609
- ARKEL Y S ET AL: "The use of coagulation activation markers (soluble fibrin polymer, TpPTM, prothrombin fragment 1.2, thrombin-antithrombin, and D-dimer) in the assessment of hypercoagulability in patients with inherited and acquired prothrombotic disorders" BLOOD COAGULATION AND FIBRINOLYSIS, vol. 13, no. 3, avril 2002 (2002-04), pages 199-205, XP009075287 ISSN: 0957-5235

## Description

La présente demande a pour objet un procédé et un test pour la détection de l'activation de la coagulation, en particulier lorsqu'elle est liée à des maladies veineuses thromboemboliques, mettant en oeuvre le dosage des D-dimères et le dosage de la fibrine soluble produits lors d'un processus d'activation de la coagulation dans le sang.

La fibrinolyse est le processus de dégradation de la fibrine dans le sang. La fibrinolyse est impliquée dans de nombreux processus physiopathologiques et se déclenche dans des situations où l'activateur tissulaire du plaminogène (t-PA) et le plasminogène se lient à la fibrine, formant un complexe ternaire fibrine-plasminogène au sein duquel le t-PA a beaucoup d'affinité pour le plasminogène, entraînant la génération de plasmine, enzyme qui dégrade la fibrine en D-dimères. En l'absence de fibrine, le t-PA a peu d'affinité pour le plasminogène, expliquant que le fibrinogène circulant n'est pas dégradé.

La dégradation de la fibrine ou fibrinolyse, conduit à la formation de produits de dégradation comprenant notamment les fragments « D-dimères ». Ces D-dimères sont associés avec le fragment E de dégradation d'une autre molécule de monomère de fibrine, formant le complexe DDE, mais même sous cette forme, sont appelés couramment D-dimères.

La fibrine soumise au processus de fibrinolyse a été préalablement formée par la conversion du fibrinogène sous l'action d'une enzyme de la coagulation, la thrombine. Lors de l'activation de la coagulation, la thrombine générée induit donc la formation de dépôts de fibrine qui constitueront le thrombus et la formation de fibrine soluble. Pour ce faire, la thrombine attaque 4 liaisons peptidiques du fibrinogène situées respectivement sur les 2 chaînes A alpha et les 2 chaînes B béta, entraînant la libération de 2 fibrinopeptides A à partir des 2 chaînes A alpha et la libération de 2 fibrinopeptides B à partir des chaînes B béta aboutissant à la formation de monomères de fibrine qui polymérisent spontanément sous la forme d'un polymère grâce à des liaisons hydrogènes établies par interaction entre les sites de polymérisation A et B démasqués lors de la libération des fibrinopeptides A et B et les sites a et b qui sont disponibles respectivement à l'extrémité des chaînes gamma et béta. Le polymère de fibrine est ensuite immédiatement stabilisé par le facteur XIII(a). La génération de thrombine est beaucoup plus importante lors de tests in vitro que celle qui a lieu in vivo. De ce fait, la génération de monomères de fibrine est beaucoup moins rapide dans le processus d'activation de la coagulation *in vivo* que dans celui généré *in vitro,* ce qui conduit une partie des monomères formés à polymériser pour donner la fibrine insoluble constituant le thrombus et une autre partie de ces monomères à réagir avec du fibrinogène dans lequel les sites a et b sont accessibles, ou encore avec des produits de dégradation du fibrinogène pour donner de la fibrine soluble dans laquelle des monomères de fibrine sont associés avec du fibrinogène.

La détermination de la concentration de fibrine soluble présente un intérêt pour témoigner de l'activation de la coagulation chez un patient. Cette détermination peut être effectuée à partir d'échantillons de sang ou de plasma obtenus à partir d'un prélèvement sanguin chez un patient.

On a ainsi montré que le dosage de la fibrine soluble est un complément utile au dosage des produits de dégradation par la fibrinolyse, puisque la fibrine soluble permet la détection d'une activation de la coagulation en cours, alors que la concentration de D-dimères témoigne d'une dégradation d'un thrombus, même si le processus d'activation de la coagulation est arrêté.

En résumé, la teneur plasmatique en D-dimères est augmentée tant que le caillot de fibrine se dégrade *in vivo.* Ainsi, si le thrombus est présent et en cours de dégradation, le taux de D-dimères est élevé, que la coagulation persiste ou soit arrêtée. Au contraire, le taux de fibrine soluble est élevé exclusivement si la coagulation persiste.

La mesure spécifique de la teneur plasmatique en fibrine soluble par rapport à la teneur en D-dimères permet donc, d'une part de déterminer la coagulation en cours chez un patient au moment où est effectué le prélèvement à analyser et d'autre part d'évaluer la balance coagulolytique.

La détermination du taux de D-dimères sur l'échantillon prélevé, dit taux de base, est ainsi le reflet de la dégradation du thrombus qui se produit *in vivo* alors que la détermination du taux de D-dimères obtenus après l'addition exogène d'un agent thrombolytique spécifique de la fibrine , représente la somme des D-dimères de base et des D-dimères provenant de la dégradation de la fibrine soluble, encore appelée fibrine circulante.

La demande de brevet WO 02/18628 décrit un procédé pour le dosage de la fibrine soluble dans un échantillon sanguin, nécessitant la mise en contact du plasma avec un activateur du plasminogène ayant un forte affinité pour la fibrine soluble (PA-Fb sp), suivie de la détermination de la teneur en produits de dégradation de la fibrine (D-dimères). La différence entre la concentration de D-dimères sur l'échantillon traité par du PA-Fb sp de celle en D-dimères de base, déterminée sur le plasma non traité par le PA-Fb sp représente donc les D-dimères liés à la dégradation de la fibrine soluble. Les inventeurs ont aujourd'hui observé que la méthode proposée dans la demande internationale antérieure WO 02/18628 peut avantageusement être complétée pour être mise en oeuvre dans le cadre de protocoles de diagnostic de maladies veineuses thromboemboliques, ainsi que dans le diagnostic et le suivi des coagulations intra vasculaires disséminées (CIVD). Elle permet aussi de déterminer si un traitement anticoagulant est efficace.

Brimble KS. et al (Thrombosis Research 88 (1997) 291-297) and Ota S. et al (American journal of Hematology 7::274-280 (2005)) disclose methods for the detection of D-Dimer and soluble fibrin in the context of deep venous thrombosis and/or pulmonary embolism.

Les maladies veineuses thromboemboliques comprennent principalement les thromboses veineuses des membres et l'embolie pulmonaire, cette dernière résultant d'une complication des susdites thromboses. D'autres thromboses veineuses que celles des membres sont néanmoins rencontrées, dans la mesure où tous les territoires veineux peuvent subir une thrombose. On citera notamment les veines rénales et mésentériques parmi celles qui figurent à l'origine de pathologies. Les maladies thromboemboliques telles que la Thrombose Veineuse Profonde (TVP) et/ou l'Embolie Pulmonaire (EP) sont des maladies affectant le pronostic vital des patients et représentant une grande proportion des invalidités et décès dans les pays industrialisés, et l'établissement d'un diagnostic de ces maladies rend aujourd'hui indispensable de compléter l'investigation par des examens d'imagerie tels que l'ultra sonographie pour le diagnostic des thromboses veineuses et la scintigraphie ou l'angiographie pour le diagnostic des embolies pulmonaires. Ces méthodes exploratoires sont d'une mise en oeuvre délicate et ne peuvent pas toujours être effectuées dans des conditions de rapidité satisfaisantes.

En conséquence, le besoin persiste, de définir un test permettant le diagnostic rapide de la maladie thromboembolique chez un patient, ce diagnostic incluant la possibilité d'exclure ladite maladie sans nécessairement avoir recours à des investigations complémentaires.

Dans le domaine du diagnostic des pathologies liées à la coagulation, on connaît la capacité des D-dimères à être utilisés si leur taux est normal, comme un indicateur négatif de la thrombose puisque l'on admet le principe selon lequel la formation d'un thrombus implique à la fois l'activation de la coagulation et de la fibrinolyse.

Cependant, le seul taux de D-dimères mesuré jusqu'à présent manque de spécificité, et ne permet pas de conclure de façon certaine qu'un thrombus intra-vasculaire soit formé, car les D-dimères présents dans la circulation peuvent provenir de la dégradation de dépôts de fibrine extra vasculaire. Les D-dimères formés « in situ » peuvent alors passer dans la circulation, d'où un taux élevé de D-dimères circulants. Dans le cadre de la présente invention, les inventeurs ont évalué la pertinence du diagnostic basé sur le test des D-dimères en association avec un test rapide pour la détermination de la fibrine soluble laquelle est représentative de l'activation de la coagulation intra-vasculaire. Cette combinaison de dosages s'est révélée présenter un intérêt dans le cadre du diagnostic de la Thrombose Veineuse Profonde (TVP) et/ou de l'Embolie Pulmonaire (EP) ainsi que dans le cadre du diagnostic de la coagulation intra vasculaire disséminée (CIVD). Le test de la fibrine soluble mis en oeuvre, appelé FSD (ou SDF en anglais) signifiant Fibrine Soluble Dégradable, permet de doser la fibrine soluble en évaluant les produits de dégradation générés lors de sa dégradation par l'activateur tissulaire du plasminogène (ou un autre agent thrombolytique tel qu'un activateur du plasminogène spécifique de la fibrine) apporté de façon exogène à un échantillon prélevé.

L'invention a donc pour objet une méthode de diagnostic *in vitro* de l'activation de la coagulation liée à un risque de maladie thromboembolique, à partir d'un même échantillon sanguin prélevé chez un patient, comprenant :
i) la mesure de la quantité de produits de dégradation de la fibrine contenus dans l'échantillon testé, consistant en la mesure de la quantité de D-dimères présents dans l'échantillon et constituant le taux de D-dimères de base ;
ii) le traitement de l'échantillon par incubation avec un activateur de plasminogène de forte affinité pour la fibrine (Pa-Fb sp) dans des conditions permettant la dégradation de la fibrine soluble contenue dans l'échantillon, en produits de dégradation sans conduire à la dégradation du fibrinogène, et la mesure de la quantité de D-dimères contenus dans l'échantillon ainsi traité ;
iii) le calcul de la différence entre la quantité de D-dimères, mesurée après l'activation par l'activateur Pa-Fb sp à l'étape (ii) et la quantité de D-dimères avant ladite activation mesurée à l'étape (i), ladite différence constituant le taux de dégradation de la fibrine soluble (SDF) ;
iv) la comparaison du taux de D-dimères mesuré à l'étape (i) avec une valeur seuil normale déterminée de ce même produit de dégradation et la comparaison du taux de SDF calculé à l'étape (iii) avec une valeur seuil normale déterminée de SDF ;
le risque de maladie thromboembolique existant si l'un au moins du taux de D-Dimères mesuré à l'étape (i) ou du taux de SDF calculé est supérieur à la valeur normale respective déterminée et l'exclusion dudit risque résultant lorsque le taux de D-Dimères obtenu à l'étape (i) et le taux de SDF obtenu à l'étape (iii) sont inférieurs aux valeurs seuils normales respectives.

La méthode de diagnostic selon l'invention est applicable pour le diagnostic de la coagulation dans le sang, que le processus de coagulation soit localisé (comme les thromboses veineuses profondes) ou généralisé (comme dans le cas des CIVD).

Le cas échéant, avant d'ajouter l'activateur de plasminogène selon l'étape a)ii) ci-dessus, on ajoute un mélange d'acide citrique et de citrate sodique dans la partie de l'échantillon sur laquelle on détermine les produits de dégradation de la fibrine soluble.

Le réactif utilisé pour doser les produits de dégradation est choisi pour mesurer un groupe donné de produits de dégradation. Par exemple, on utilise des anticorps de spécificité déterminée vis à vis d'un type particulier de produits de dégradation de la fibrine.

Lorsque la concentration de D-dimères de base mesurée à l'étape i) a une valeur supérieure à la valeur seuil de 500 ng/ml, le taux de D-dimères est considéré comme augmenté. Lorsque la concentration de D-dimères correspondant à la dégradation de la fibrine soluble et qui est calculée à l'étape iii) a une valeur supérieure à la valeur seuil de 300 ng/ml déterminée chez les sujets sains, elle est considérée comme augmentée.

Ces valeurs seuils ont été déterminées avec un réactif constitué par un anticorps du test Lia-test de la société Diagnostica Stago ou du test VIDAS de la société Bio-Mérieux. Pour d'autres réactifs, la valeur seuil devrait être déterminée par comparaison avec le résultat obtenu avec les susdits réactifs.

Lorsque les mesures des D-dimères d'une part, de Fibrine Soluble Dégradable d'autre part, ont été effectuées, on considère que chez le patient dont l'échantillon sanguin a été testé, il existe un risque de maladie thromboembolique lorsque le taux de (D-dimères) produits par la dégradation de la fibrine de base est supérieur ou égal à 500 ng/ml ou lorsque le taux de fibrine soluble évaluée par la différence entre le taux de D-dimères présent dans le plasma traité par l'activateur du plasminogène fibrine spécifique et celui du taux de D dimères de base est supérieur à la valeur seuil, par exemple 300 ng/ml.

L'échantillon biologique est de préférence un liquide biologique, par exemple un échantillon de sang ou de plasma, ou encore un liquide de ponction, à condition que la teneur en plasminogène dans ce liquide soit identique à celle du plasma. Au cas où les liquides de ponction renferment peu de plasminogène, il faudrait envisager l'apport de Glu-plasminogène de manière à ce que la concentration en plasminogène devienne voisine de celle du plasma.

L'activateur du plasminogène de forte affinité pour la fibrine (i.e. qui n'active le plasminogène qu'au sein de la fibrine) utilisé dans la méthode de dosage de la fibrine soluble par génération de produits de dégradation spécifiques peut être choisi parmi les nombreux composés connus comme activateurs du plasminogène. Certains toutefois dégradent aussi bien le fibrinogène que la fibrine, tels par exemple la streptokinase et l'urokinase. Ces composés ne sont pas adaptés à la méthode de l'invention car ils conduisent à une dégradation du fibrinogène donnant lieu à des produits de dégradation du fibrinogène qui interfèrent avec ceux résultant de la dégradation de la fibrine.

Un autre groupe d'activateurs du plasminogène est constitué par des composés décrits comme présentant une forte spécificité pour dégrader la fibrine, par rapport au fibrinogène. La méthode de l'invention utilise avantageusement la spécificité de cet autre groupe de composés pour sa mise en oeuvre, et fait appel par exemple à
◆ l'activateur tissulaire du plasminogène (ou t-PA) ou ses dérivés comme, par exemple, le TNK-tPA qui est un mutant du t-PA qui a une très grande spécificité pour la fibrine (Cannon CP, et al "TNK-tissue plasminogen activator compared with front-loaded altephase in acute myocardial infarction results of the TIMI 10B trial". Thrombolysis in Myocardial Infarction (TIMI) 10B Investigators, Circulation 98 (25), 2805-14, 1998.) ;
◆ l'activateur provenant de la salive de Desmodus rotundus (bat-tPA ou vPA = Vampire bar salivary plasminogen activator) ou ses dérivés :
   DSPAs = Desmodus rotundus salivary PAs, FEKP = DSPA alpha 1 et alpha 2, EKP = DSPA beta, KP = DSPA gamma, (Bringmann P et al. : "Structural features mediating fibrin selectivity of vampirebat plasminogen activators". J Biol Chem, 270, 25596-603, 1995.).
      la Staphylokinase, (SAK), polypeptide sécrété par le Staphylococcus aureus (Collen D. : "Staphylokinase : a potent, uniquely fibrin-selective thrombolytic agent". Nat Med, 4 - 279-84, 1998.sakharov DV et al: "Fibrin-specificity of a plasminogen activator affects the efficiency of fibrinolysis and responsiveness to ultrasound : comparison of nine plasminogen activators in vitro". Thromb Haemos, 81, 605-12, 1999.) ou un de ses mutants (Collen D et al: "Recombinant staphylokinase variants with altered immunoreactivity. I : Construction and characterization". Circulation, 94, 197-206, 1996).

Pour la réalisation de la méthode de diagnostic précédemment décrite, on utilise des anticorps anti-D-dimères, pour effectuer les deux dosages (D-dimères de base et D-dimères après action de l'activateur du plasminogène Fibrine spécifique) de la méthode de l'invention. De tels anticorps ont été décrits dans l'art antérieur et sont également commercialisés par exemple par la société Diagnostica Stago sous le nom Lia-test ou sous le nom Vidas par la société Bio-Mérieux.

Pour pouvoir être comparés, les dosages des étapes i) et ii) doivent faire intervenir le même anticorps anti-D-dimères.

Les D-Dimères résultant de la dégradation de la fibrine soluble en présence de PA Fb sp, peuvent être dosés selon toute technique courante de dosage d'analytes telles que par exemple des méthodes de type ELISA, des méthodes sensibles par agglutination de billes de latex (du type de celles utilisées dans le Liatest), des méthodes d'immunochromatographie, etc. Parmi les différents tests de dosage des D-Dimères disponibles dans le commerce, on peut citer par exemple l'ASSERACHROM D-Di ou le STA LIATEST D-Di, tous deux commercialisés par Diagnostica Stago. Toutefois, dans le cadre de la présente invention, les conditions d'utilisation du test ELISA de l'ASSERACHROM D-Di ont été avantageusement modifiées pour raccourcir le test (15 min. d'incubation avec l'anticorps immobilisé et 15 minutes avec l'anticorps marqué à la peroxydase).

Avantageusement, la méthode de diagnostic *in vitro* selon l'invention comprend en outre le traitement d'un échantillon contrôle positif, en particulier d'un plasma contrôle positif.

Pour l'obtention du plasma contrôle positif, le plasma est d'abord incubé avec de la thrombine en faible quantité pendant un temps déterminé pour permettre la formation de fibrine soluble, sans la formation de caillot de fibrine. Le processus de coagulation qui a été ainsi déclenché est ensuite bloqué par l'addition d'un inhibiteur de la thrombine pour éviter que la réaction ne se poursuive. On utilise par exemple l'hirudine ou l'héparine en tant qu'inhibiteur.

Les temps d'incubation du plasma et les concentrations en thrombine et en inhibiteur pour le blocage sont avantageusement déterminés de façon à obtenir une activation de la coagulation conduisant à la génération de fibrine soluble sans qu'il y ait formation d'un caillot de fibrine.

L'incubation en présence d'activateur de la coagulation (thrombine) est de préférence réalisée pendant un temps d'incubation de 2 minutes, à température ambiante. L'inhibiteur est ensuite ajouté en large excès pour être sûr de bloquer la coagulation.
◆ S'il s'agit de l'hirudine, celle-ci est avantageusement utilisée à une concentration finale de 100 µg/ml pour une concentration finale en thrombine de 0,18 U/ml.
◆ S'il s'agit d'héparine, celle-ci est utilisée à 500 U/ml en concentration finale lorsque la concentration finale en thrombine utilisée est de 0,18U/ml.

Selon un autre mode de réalisation avantageux de l'invention, la méthode de diagnostic *in vitro* comprend aussi le traitement d'un échantillon contrôle négatif, en particulier d'un plasma contrôle négatif. La description de la préparation de ces contrôles est donnée dans les exemples avec des détails supplémentaires.

L'évaluation de la fibrine soluble selon la présente invention met en oeuvre une première étape de dégradation de la fibrine soluble par le PA Fb sp, suivie d'une mesure des produits de dégradation spécifiques résultant de l'action du PA Fb sp.

Il est indispensable que les résultats de la méthode selon l'invention puissent être obtenus le plus rapidement possible, tout en étant représentatifs de la quantité de fibrine soluble présente dans l'échantillon. Pour ce faire, les conditions d'utilisation du PA Fb sp doivent être déterminées de façon à ce que la dégradation de la fibrine soluble soit rapide et qu'elle ne s'accompagne pas d'une dégradation "contaminante" du fibrinogène plasmatique circulant, donnant lieu à des produits de dégradation interférant avec ceux provenant de la fibrine soluble dans le dosage.

Les doses de PA Fb sp à utiliser et le temps d'incubation avec le plasma sont donc choisis de façon à induire l'augmentation du taux de produits de dégradation de la fibrine la plus importante dans les témoins positifs et une augmentation pratiquement nulle dans les témoins négatifs (i.e. n'ayant pas subi de traitement par un activateur de la coagulation).

Différents activateurs de la fibrinolyse permettant la dégradation spécifique de la fibrine soluble peuvent être utilisés dans le cadre de la présente invention. Avantageusement, le PA Fb sp est choisi au sein du groupe constitué par les activateurs cités précédemment, à savoir: le tPA ou ses dérivés, le VPA ou ses dérivés, la staphylokinase ou un de ses mutants. On utilise de préférence le tPA ou la Staphylokinase, et plus préférentiellement, le tPA.

Dans des conditions d'incubation des échantillons de 15 minutes à 37° C, la concentration finale de staphylokinase testée est comprise entre 1 et 12 µg/ml. La concentration finale retenue est avantageusement de 10 µg/ml. Le temps d'incubation peut être modifié et sa variation sera déterminée en fonction de la nature et de la concentration de la PaFbsp utilisée.

Le tPA est avantageusement utilisé dans une gamme de concentrations finales comprise entre 1 et 2,5 µg/ml. Préférentiellement, le tPA est utilisé à 2 µg/ml pendant un temps d'incubation de 15 minutes à 37°C.

Selon un mode de réalisation particulier de l'invention, la dégradation de la fibrine soluble par l'activateur de plasminogène, sans dégradation du fibrinogène, peut être bloquée après dégradation de la fibrine soluble en ajoutant un inhibiteur de la plasmine, par exemple l'aprotinine. Des caractéristiques particulières pour l'utilisation de l'aprotinine, ou de façon équivalente d'un autre inhibiteur de la plasmine, sont données dans les exemples. La quantité utilisée d'aprotinine est par exemple équivalente à la quantité utilisée d'activateur du plasminogène. Ainsi, l'inhibiteur de la plasmine est ajouté après 15 minutes d'incubation à 37°C avec l'activateur du plasminogène.

Dans un mode de réalisation particulier de l'invention, on peut ajouter, préalablement à l'addition de l'activateur de plasminogène, un anticoagulant tel qu'une solution contenant de l'acide citrique et du citrate sodique, à la fois à l'échantillon dosé et dans les échantillons contrôles. Les quantités et modalité d'ajout de l'acide citrique et du citrate sodique sont indiquées dans les exemples.

Dans le cadre de la présente invention, la méthode de diagnostic décrite précédemment est appliquée à la recherche de la formation d'un thrombus veineux.

Selon une application particulière de la méthode de diagnostic de l'invention, le procédé est mis en oeuvre pour le diagnostic d'exclusion d'une thrombose veineuse profonde.

Selon un mode de réalisation particulière de l'invention, la méthode de diagnostic est mise en oeuvre pour le diagnostic d'exclusion d'une embolie pulmonaire.

Selon l'invention, la méthode est mise en oeuvre sur un échantillon sanguin prélevé chez un patient préalablement à la mise en oeuvre d'un traitement anticoagulant.

En principe, le dosage de la fibrine soluble pour le diagnostic d'exclusion d'une thrombose veineuse doit être effectué avant tout traitement anticoagulant. En effet, si le malade est soumis à un traitement au moyen d'anticoagulants, la concentration en fibrine soluble diminue très vite pour atteindre des valeurs normales. Chez les malades traités, la détermination de la concentration en fibrine soluble dans le plasma permet seulement de déterminer si l'anticoagulation est efficace.

Selon un mode de réalisation particulier de la méthode, le dosage de la fibrine soluble est effectué en utilisant comme activateur de plasminogène le tPA.

D'autres caractéristiques de l'invention apparaissent dans les exemples qui suivent et les figures.

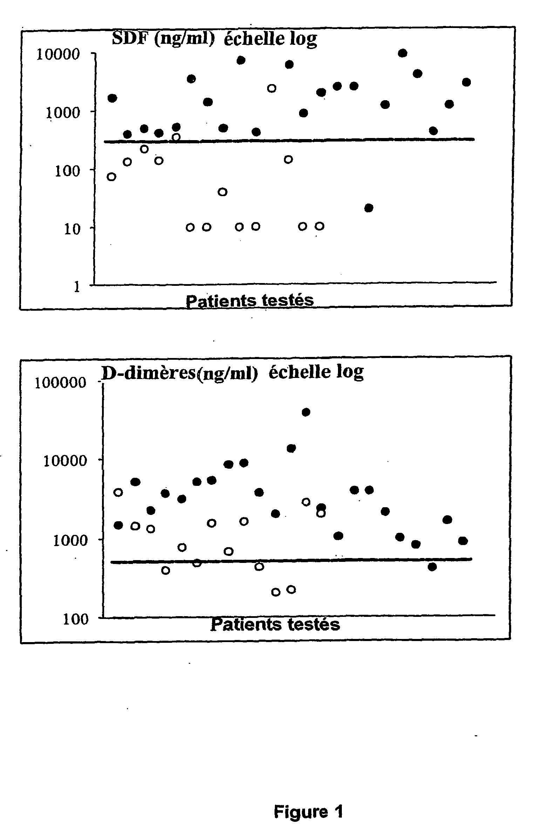

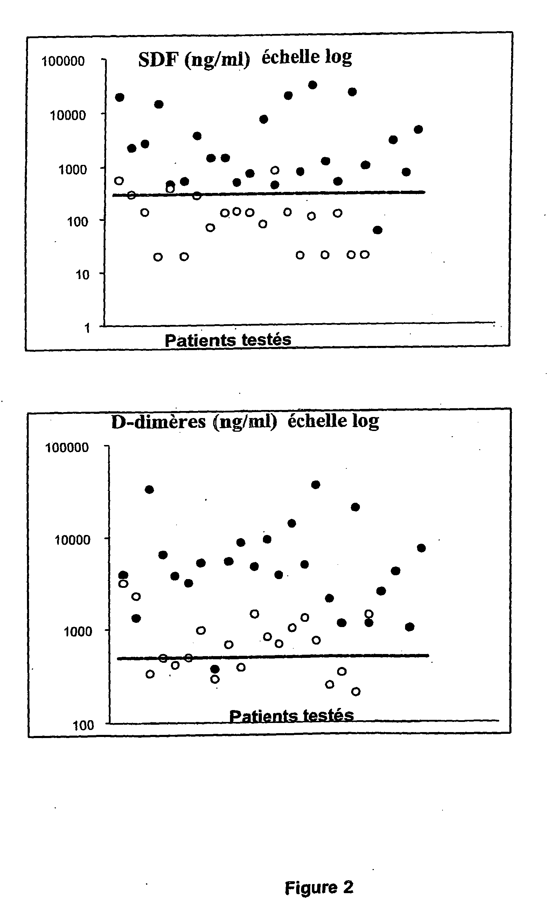

Les figures 1 et 2 représentent respectivement la comparaison des D-dimères et des SDF chez des patients avec suspicion d'embolie pulmonaire ou suspicion de thromobose veineuse profonde. Dans chaque cas, les cercles noirs correspondent aux patients affectés et les cercles blancs aux patients normaux. La ligne donne la limite supérieure de la valeur normale

### EXEMPLES

### Exemple n°1 :

### Choix de la concentration de thrombine utilisée pour l'obtention d'un plasma contrôle positif renfermant de la fibrine soluble :

Le plasma contrôle positif est préparé selon le protocole suivant :

| | |
|---|---|
| Plasma normal | 200 µl |
| Thrombine humaine (Stago réf. 00896) 0,5 à 1 U/ml (selon le plasma utilisé) | 20 µl |
| Incubation de 2 min. à la température du laboratoire. | |
| Hirudine (Knoll) | 100µg/ml (concentration finale) |
| ou | |
| Héparine (Choay) | 5000 UI/ml (concentration finale) 20 µl |

Vérifier :
- qu'il n'y a pas eu de formation de caillot dans le tube.
- qu'un test de détection de la fibrine soluble disponible dans le commerce soit positif (ex. FS test du laboratoire Stago).

| | **Contrôle positif** | **Contrôle négatif** |
|---|---|---|
| Plasma | 200 µl | 200 µl |
| Thrombine 1 U/ml ou 0.5 U/ml | 20 µl | - |
| Sérum physiologique | - | 20 µl |

| **2 min (+/- 10 sec) à température ambiante** | | |
|---|---|---|
| Héparine à 5000UI/ml | 20µl | - |
| Sérum physiologique | - | 20 µl |

### Exemple n° 2 :

### Détermination de la quantité de PA Fb sp à utiliser dans des conditions d'incubation définies.

Pour réaliser la méthode de l'invention, la quantité d'activateur à ajouter à l'échantillon testée doit être telle qu'elle induise une génération des D-dimères importante dans le plasma contrôle positif, tel qu'obtenu dans l'exemple n° 1, et une génération des D-dimères non significative dans un plasma contrôle négatif (témoin non traité par la thrombine).

Une incubation des plasmas témoins et des plasmas contrôles positifs (n = 21) a donc été réalisée avec différentes doses de PA Fb sp pendant 15 minutes à 37° C. A la fin du temps d'incubation, les D-dimères sont déterminés par Liatest ou par Elisa rapide (D-Di Stago) (incubation de 15 minutes à 37° C avec l'anticorps de capture et de 15 minutes à 37° C avec l'anticorps de révélation).

Les résultats présentés dans le tableau Il ont été obtenus avec le test ELISA.

Des résultats sensiblement analogues ont été obtenus avec le Liatest (n = 5).

**Tableau II : Dégradation de la fibrine soluble par des quantités croissantes de t-PA et de SAK.**

| | **D- dimères (ng/ml)** | **Fibrine* soluble (ng/ml)** | **D- dimères (ng/ml)** | **Fibrine* soluble (ng/ml)** |
|---|---|---|---|---|
| | Contrôle négatif | | Contrôle positif | |
| Sans addition de PA Fb sp | 375 | | 375 | |
| Après Staphylokinase (µg/ml) | | | | |
| 10 | 400 | < 50 | 1750 | 1375 |
| 2 | 390 | < 50 | 1615 | 1240 |
| 1,5 | 375 | < 50 | 1700 | 1325 |
| 1 | 350 | < 50 | 1657 | 1282 |
| 0,5 | 410 | < 50 | 1125 | 750 |
| Après t-PA (µg/ml) | | | | |
| 2 | 350 | < 50 | 1790 | 1415 |
| 1 | 360 | < 50 | 1420 | 1045 |
| 0,5 | 360 | < 50 | 1210 | 835 |

| | | | | |
|---|---|---|---|---|
| * Fibrine soluble = taux de D-dimères après l'addition de tPa ou de Staphylokinase - taux de D-dimères de base avant l'addition de tPa ou de Staphylokinase. | | | | |

La dose de PA Fb sp choisie est celle qui induit :
- une augmentation < 300 µg/ml dans les plasmas contrôles non traités (témoins négatifs),
- l'augmentation la plus importante dans les plasmas contrôles positifs.

De ces résultats, il ressort que les concentrations finales préférées de PA Fb sp à utiliser sont :
- 2 µg/ml pour le t-PA : dans ces conditions, la dose de t-PA susceptible d'être neutralisée par les PAI (inhibiteurs des activateurs du plamsinogène)est négligeable,
- 10 µg/ml pour la SAK (des doses inférieures de SAK ont induit une mauvaise dégradabilité de la fibrine soluble chez certains malades ou certains contrôles positifs, vraisemblablement du fait de la présence d'anti-staphylokinase dans le prélèvement, anti-staphylokinase qui peuvent apparaître, suite à une infection par des staphylocoques).

### Exemple n° 3 : Dosage des D-dimères et de la fibrine soluble.

Dans l'investigation conduite, le dosage des D-dimères et celui de la fibrine soluble ont été effectués chez 87 patients consécutifs, consultant dans les unités d'urgence pour une suspicion de thrombose veineuse et/ou d'embolie pulmonaire et qui n'avaient reçu aucun traitement. Chez ces malades ont été réalisés un doppler pour le diagnostic des thromboses veineuses profondes, une scintigraphie ou une angiographie pulmonaire pour le diagnostic d'embolie pulmonaire. Les taux de D-dimères et de Fibrine Soluble Dégradable (FSD) ont été déterminés avant le début du traitement anticoagulant. Il a été montré que la sensibilité du dosage de la fibrine soluble dégradable est similaire à celle du dosage des D-dimères (96% pour les D-dimères et pour la fibrine soluble dégradable). De façon intéressante, les « faux négatifs » pour les taux de D-dimères et de fibrine soluble dégradable ont été observés chez des patients différents. Dès lors, l'association des deux tests peut augmenter la sensibilité du diagnostic de la thrombose (100%). En outre, la spécificité de la fibrine soluble dégradable dans le diagnostic de la maladie veineuse thromboembolique est plus importante (86% et 87 % respectivement pour l'embolie pulmonaire et pour la thrombose veineuse profonde) que celle des D-dimères (36% et 42% respectivement pour l'embolie pulmonaire et pour la thrombose veineuse profonde).

Une normalisation rapide de la fibrine soluble dégradable a été observée chez les patients sous traitement anticoagulant à dose curative. Dès que le traitement anticoagulant est institué, le taux de fibrine soluble chute. En conséquence, la fibrine soluble dégradable ne peut pas être utilisée comme test de diagnostic chez des patients déjà traités par des anticoagulants. Cependant, la fibrine soluble dégradable peut être utile pour le suivi du traitement anticoagulant. En conclusion, il est indiqué que le taux de fibrine soluble dégradable en association avec celui des D-dimères est un outil clinique utile pour prédire ou pour exclure une embolie pulmonaire et/ou une thrombose veineuse profonde.

La fibrine soluble est présente lors de l'activation de la coagulation. Son augmentation est observée dès les stades précoces de cette activation.

Dans l'art antérieur, plusieurs tests ont déjà été développés pour évaluer la fibrine soluble chez des patients présentant une thrombose mais, du fait de la variabilité des résultats dans les essais disponibles, l'intérêt de la détermination de la fibrine soluble dans le diagnostic d'exclusion de la maladie veineuse thromboembolique n'a pas été établi (1-21).

Le but de la présente étude était d'évaluer l'utilité potentielle d'un nouveau test, basé sur la détermination du taux de Fibrine Soluble Dégradable, simple, sensible et rapide et hautement spécifique des polymères de fibrine soluble plasmatique.

Ce test est basé sur l'évaluation des D-dimères générés après incubation du plasma avec du t-PA dans des conditions induisant la dégradation de la fibrine soluble mais n'induisant pas la dégradation de fibrinogène plasmatique. Ainsi, cet essai a été appelé Fibrine Soluble Dégradable (SDF). En fait, en dépit du petit nombre de monomères de fibrine dans la fibrine soluble, les monomères de fibrine sont réticulés ensemble parce que l'activation du facteur XIII coïncide avec le relargage du fibrinopeptide A, et en plus l'activation du facteur XIII par la thrombine est accélérée par la présence de fibrine (22). Parmi les marqueurs de la génération de thrombine *in vivo*, tel que le fibrinopeptide A (FPA, demi-vie 3 minutes (23) ou le complexe thrombine anti-thrombine (TAT, demi-vie 15 minutes) (24), la Fibrine Soluble Dégradable a été choisie parce que sa mesure peut être plus sensible parce qu'elle est ainsi moins sensible aux artéfacts de mesures.

La présente étude a été réalisée pour évaluer la performance du diagnostic basé sur la combinaison des taux de D-dimères et de fibrine soluble dégradable, chez des patients consécutifs non traités avec suspicion clinique d'embolie pulmonaire (n = 38) ou de thrombose veineuse profonde (n = 49) inscrits dans les unités d'urgence de 3 centres différents. En cas de suspicion de thrombose veineuse profonde, le diagnostic a été confirmé par examen de la compression par ultra-sons, le diagnostic de l'embolie pulmonaire ayant été confirmé soit par scintigraphie soit par angiographie pulmonaire. La valeur seuil de fibrine soluble dégradable pour un test considéré comme positif était 300 ng/ml.

Dans cette étude, dans le but d'analyser les effets de l'anticoagulation, les profils d'évolution de la fibrine soluble dégradable et des D-dimères ont été également examinés chez les patients souffrant d'embolie pulmonaire et/ou de thrombose veineuse profonde, après le début du traitement anticoagulant, pour tester l'efficacité de la thérapeutique dans la maladie thrombo-embolique.

### Matériel et méthodes

Echantillons de plasma : le sang a été collecté sur 0,13 M de citrate (1 part de citrate pour 9 parts de sang). Après centrifugation à 2500g pendant 15 minutes, les plasmas ont été collectés et congelés à - 20°C jusqu'à leur utilisation.

Cependant, lorsque le taux de fibrine soluble dégradable est très élevé, comme en cas de coagulation intra-vasculaire (CIVD), la fibrine soluble dégradable peut former un complexe insoluble, durant les étapes de congélation et de décongélation, et par conséquent, il est recommandé de réaliser ce test avec du plasma fraîchement collecté.

Le sang a été obtenu à partir de volontaires sains ou des patients extérieurs s'étant présentés dans les unités d'urgence. Les patients ayant reçu un traitement anticoagulant ont été considérés uniquement pour le suivi. La population des patients consistait en des patients consécutifs présentant des signes cliniques d'embolie pulmonaire ou de thrombose veineuse profonde, qui ont été diagnostiqués par analyse en ultrasonographie de la compression des veines proximales de jambes, par scintigraphie pulmonaire et par angiographie pulmonaire, pour vérifier le diagnostic.

### Tests biologiques

### Détermination des D-dimères

La mesure des D-dimères est réalisée par agglutination de micro-particules de latex revêtues avec des anticorps monoclonaux contre les D-dimères utilisant le Lia-test (Diagnostica Stago) dans un appareil STA, ou par ELISA utilisant VIDAS (Bio-Mérieux).

### Mesure de la fibrine soluble dégradable : réalisée en 3 étapes

1-la dégradation de la fibrine : à 200 µl de plasma on a ajouté 20 µl de t-PA à 20µg/ml (plasma traité) ou 20 µl de sérum physiologique (plasma non traité). Après 15 minutes d'incubation à 37°C, la plasmine générée est bloquée par addition de 20 µl d'aprotinine (Pentapharm) à 12,5 TIU/ml.
2-La concentration en D-dimères a alors été déterminée par le test « D-dimers Liatest » de Diagnostica Stago.
3-Les taux de fibrine soluble dégradable ont été calculés comme la différence entre la concentration en D-dimères dans du plasma traité et celle présente dans du plasma non traité.

Lorsque le taux de D-dimères dans le plasma était supérieur à 4000 ng/ml, l'échantillon était dilué après l'étape de dégradation.

La fibrine soluble utilisée comme contrôle positif a été obtenue par l'incubation de plasma normal avec des faibles doses de thrombine pendant un temps court au bout duquel la thrombine est bloquée par l'héparine.

Le plasma de l'échantillon à tester, mais aussi le contrôle positif et le contrôle négatif pour le dosage de la fibrine soluble sont dosés de la manière indiquée dans le tableau III.

**Tableau III**

| | D-dimères de base | SDF+ D-dimère de base |
|---|---|---|
| Plasma | 200 µl | 200 µl |
| AC pur | 20µL | 20µl |
| tPA | - | 20µL |
| Sérum physiologique | 40µL | - |

| 15 min à + 37°C | | |
|---|---|---|
| Aprotinine | - | 20µl |

L'addition d'aprotinine permet de bloquer la plasmine à un temps déterminé conduisant à la dégradation de la fibrine seule et non du fibrinogène.
- Le contrôle positif et le contrôle négatif sont reconstitués avec de l'ACd, c'est-à-dire de l'AC dilué au 1/5.

### Préparation de l'AC :

| | | |
|---|---|---|
| Acide citrique, H2O | PM=210.14 | 0.16 g |
| Citrate trisodique, 2H2O | PM=294.10 | 0.44 g |
| H20 | | 20 ml |

### Résultats

### Spécificité des mesures

- Chez les volontaires normaux sains (n = 180), le taux de fibrine soluble était très faible, inférieur ou égal à 300 ng/ml.
- En outre, il n'y a pas de corrélation entre la concentration plasmatique en fibrine soluble et celle en D-dimères, puisqu'après traitement des malades par l'héparine, la concentration en fibrine soluble chute très rapidement alors que la concentration en D-dimères chute beaucoup plus tardivement puisqu'elle reflète la dégradation du caillot qui persiste après lé bloquage de l'activation de la coagulation.

### Taux de D-dimères et de fibrine soluble chez des patients suspectés de maladie veineuse thromboembolique ou d'embolie pulmonaire

### Chez des volontaires normaux sains (n = 180) :

La valeur moyenne était de 80 ± 106 ng/ml et parmi les 180 volontaires testés, 140 présentaient un taux de fibrine soluble dégradable indétectable. La valeur seuil prise pour un test positif était 300 ng/ml.

### Chez des patients suspectés d'embolie pulmonaire ou de thrombose veineuse profonde

Sur 38 patients présentant une suspicion d'embolie pulmonaire, 23 étaient positifs selon l'analyse en imagerie et sur 49 avec suspicion de thrombose veineuse profonde, 25 étaient positifs, conformément à l'observation d'une compression anormale des veines proximales de la jambe en ultrasonographie.

Parmi les 2 groupes de patients, ceux présentant une embolie pulmonaire (n = 23) ou une thrombose veineuse profonde (= 25), 2 présentaient des taux de fibrine soluble qui étaient des faux négatifs, un parmi le groupe présentant une embolie pulmonaire (confirmée par angiographie) et l'autre dans le groupe de patients présentant une thrombose veineuse profonde (confirmée par ultrasonographie) ; cependant, ces 2 patients ont des taux de D-dimères supérieurs à la valeur seuil de 500 ng/ml. Inversement, les taux de D-dimères étaient normaux (< 500 ng/ml) chez 2 patients (un parmi le groupe présentant une embolie pulmonaire et l'autre dans le groupe de patients présentant une thrombose veineuse profonde) alors que les taux de fibrine soluble dégradable étaient > 300 ng/ml. Ces taux de D-dimères qui sont des faux négatifs étaient observés, que le taux de D-dimères soit déterminé par le Lia test® ou par le test Vidas®.

Avec la combinaison des D-dimères et de la fibrine soluble, aucun faux négatif n'a été détecté dans les valeurs biologiques.

Les indices de pertinence (sensibilité, spécificité, valeur prédictive positive et négative) des D-dimères, de la fibrine soluble dégradable ont été calculés.
(tableau III)

**Tableau III : Sensibilité, spécificité, valeur prédictive positive et négative des D-dimères, de la fibrine soluble dégradable.**

| | SDF+ | | D-dimères | |
|---|---|---|---|---|
| | EP | TVP | EP | TVP |
| VPP | 92% | 88% | 71% | 60% |
| VPN | 92% | 95% | 83% | 90% |
| sensibilité | 96% | 96% | 96% | 96% |
| spécificité | 86% | 87% | 36% | 37% |

VPP = valeur prédictive positive
VPN = valeur prédictive négative
EP = embolie pulmonaire
TVP = thrombolie veineuse profonde
SDF= fibrine soluble dégradable

### Evolution des taux de D-dimères et de fibrine soluble dégradable chez des patients sous traitement anticoagulant.

Une normalisation rapide de la fibrine soluble dégradable a été observée chez des patients sous héparine non fractionnée ou sous héparine de bas poids moléculaire donnée à dose curative. Après le jour 1, les taux de fibrine soluble dégradable étaient normaux ou dans la limite supérieure de la valeur normale. L'analyse journalière des taux a révélé que la fibrine soluble dégradable restait dans des valeurs normales, durant le traitement sous héparine. Au contraire, les taux de D-dimères diminuaient lentement et les taux n'atteignaient pas les valeurs normales pendant le traitement sous héparine. Chez un patient, la fibrine soluble était encore augmentée au cours du traitement thérapeutique, témoignant d'un effet thérapeutique insuffisant.

### Discussion

Il existe un besoin pour un outil diagnostic non invasif et adéquat pour le diagnostic de l'embolie pulmonaire et/ou de la thrombose veineuse profonde, permettant de prendre une décision immédiate dans la plupart des cas sur le traitement à donner.

Les objectifs de cette étude étaient de déterminer si la combinaison des D-dimères et de la fibrine soluble dégradable pouvait être utile pour le diagnostic de l'embolie pulmonaire et/ou de la thrombose veineuse profonde et pour évaluer l'efficacité d'un traitement anticoagulant pour inhiber la thrombogénèse.

*Concernant la sensibilité des mesures dans l'embolie pulmonaire et de la thrombose veineuse profonde :* les 2 patients présentant des résultats faux négatifs relatifs aux taux de D-dimères (un parmi le groupe présentant une embolie pulmonaire et l'autre dans le groupe de patients présentant une thrombose veineuse profonde) avaient des taux de fibrine soluble dégradable supérieurs à la limite supérieure de la valeur seuil. Cela pourrait être dû à une structure de caillot de fibrine anormale (congénitale ou acquise) rendant les caillots anormalement résistants à la fibrinolyse. Cette anomalie pouvait directement contribuer à un risque accru de thrombose due à une thrombolyse défectueuse.

Cette hypofibrinolyse peut expliquer les 3 à 5 % de cas « faux négatifs » en D dimères chez les patients présentant une thrombose constituée. Dans la détermination de la fibrine soluble dégradable, la fibrine est facilement accessible aux enzymes fibrinolytiques. En revanche la fibrine du thrombus, quand elle est formée de fibres fines et très serrées, est peu accessible aux enzymes fibrinolytiques, expliquant les 3 à 5% de faux négatifs en D-dimères observés dans les thromboses. Les taux négatifs relatifs à la fibrine soluble dégradables ont été détectés chez 2 patients (un parmi le groupe présentant une embolie pulmonaire et l'autre dans le groupe de patients présentant une thrombose veineuse profonde) alors que les taux de D-dimères chez ces 2 patients étaient, supérieurs à 500 ng/ml. Cela pourrait suggérer que la thrombogénicité est un processus non évolutif parce que l'activité de la thrombine est transitoire.

Les inventeurs ont démontré que la combinaison des 2 tests (fibrine soluble dégradable et D-dimères) peut être utilisée pour écarter de façon certaine le diagnostic d'embolie pulmonaire et de thrombose veineuse profonde chez des patients.

Les D-dimères plasmatiques, remplissent dans la majorité des cas les critères requis pour le diagnostic de l'embolie pulmonaire et de la thrombose veineuse profonde car ce sont des marqueurs sensibles pour la thrombose mais manquent de spécificité (26-35).

Il est maintenant bien établi que les D-dimères ne peuvent être utilisés pour faire un diagnostic positif de la maladie veineuse thromboembolique car le test est peu spécifique. Cependant, lorsque le taux de D-dimères est normal, le diagnostic de maladie thrombo-embolique peut être écarté chez 95% des patients.

Dans cette étude, il a été montré que la spécificité de la fibrine soluble est plus importante que celle des D-dimères. Cette faible spécificité des mesures des D-dimères dans le diagnostic de la maladie veineuse thromboembolique peut être due à la dégradation par des enzymes locales fibrinolytiques de la fibrine présente dans les tissus. Les produits de dégradation de la fibrine soluble formés localement dans les tissus diffusent dans le sang, du fait de leur poids moléculaire relativement peu important. Cela est soutenu par l'observation que le taux de D-dimères est souvent élevé chez des patients avec des maladies inflammatoires.

Dans cette étude, il a également été montré que le taux de fibrine soluble dégradable est augmenté dans les événements thrombotiques ; cependant, aussitôt que les traitements anticoagulants sont administrés, la concentration de fibrine soluble dégradable décroît jusqu'à atteindre une valeur normale en quelques heures. Cela suggère que les médicaments anticoagulants sont capables de bloquer efficacement le processus thrombotique. La persistance de l'élévation du taux de D-dimères est due à la dégradation du thrombus formé avant le traitement anticoagulant. Par conséquent, l'intérêt de la détermination de la fibrine soluble dégradable, pour suivre l'efficacité du traitement anticoagulant ou tester l'efficacité de nouveaux médicaments anti-thrombotiques, est proposé.

En conclusion, les résultats de cette étude suggèrent que la détermination de la fibrine soluble dégradable en association avec celle des D-dimères peut être considérée comme un outil clinique utile pour le diagnostic de la thrombose veineuse profonde et l'embolie pulmonaire et également d'autres événements thrombotiques. En outre, il est suggéré que la fibrine soluble dégradable peut être utile pour le suivi des effets d'un traitement anticoagulant.

### REFERENCES BIBLIOGRAPHIQUES

1. Arkel YS, Ku DH, Le P, Carr AM. Comparison of a test for soluble fibrin polymer (TpP) with a standard quantitative ELISA for D-dimer in patients, without current thrombosis, who have cancer or renal disease. Thromb Haemost. 2001 ;86:1127-8.
2. Arkel YS, Paidas MJ, Ku DH. The use of coagulation activation markers (soluble fibrin polymer, TpP, prothrombin fragment 1.2, thrombin-antithrombin, and D-dimer) in the assessment of hypercoagulability in patients with inherited and acquired prothrombotic disorders. Blood Coagul Fibrinolysis. 2002;13 :199-205.
3. Brimble KS, Ginsberg JS. Evaluation of the combination of a bedside D-dimer assay and enzyme-linked immunosorbent soluble fibrin assay in patients with suspected venous thromboembolism. Thromb Res. 1997 ;88:291-7.
4. Dempfle CE, Dollman M, Lill H, Puzzovio D, Dessauer A, Heene DL. Binding of a new monoclonal antibody against N-terminal heptapeptide of fibrin alpha-chain to fibrin polymerization site 'A': effects of fibrinogen and fibrinogen derivatives, and pretreatment of samples with NaSCN. Fibrinolysis. 1993 ;4:79-86.
5. Dempfle CE, Pfitzner SA, Dollman M, Huck K, Stehle G, Heene DL. Comparison of immunological and functional assays for measurement of soluble fibrin. Thromb Haemost. 1995 ;74:673-9.
6. Dempfle CE, Zips S, Ergul H, Heene DL; FACT study group. The fibrin assay comparison trial (FACT): correlation of soluble fibrin assays with D-dimer. Thromb Haemost. 2001 ;86:1204-9.
7. Dempfle CE, Wurst M, Smolinski M, Lorenz S, Osika A, Olenik D, Fiedler F, Borggrefe M. Use of soluble fibrin antigen instead of D-dimer as fibrin-related marker may enhance the prognostic power of the ISTH overt DIC score. Thromb Haemost. 2004 ;91:812-8.
8. Derhaschnig U, Laggner AN, Roggla M, Hirschl MM, Kapiotis S, Marsik C, Jilma B. Evaluation of coagulation markers for early diagnosis of acute coronary syndromes in the emergency room. Clin Chem. 2002 ;48:1924-30.
9. Ginsberg JS, Siragusa S, Douketis J, Johnston M, Moffat K, Stevens P, Brill-Edwards P, Panju A, Patel A. Evaluation of a soluble fibrin assay in patients with suspected deep vein thrombosis. Thromb Haemost. 1995 ;74:833-6.
10. Ginsberg JS, Siragusa S, Douketis J, Johnston M, Moffat K, Donovan D, McGinnis J, Brill-Edwards P, Panju A, Patel A, Weitz JI. Evaluation of a soluble fibrin assay in patients with suspected pulmonary embolism. Thromb Haemost. 1996 ;75:551-4.
11. Hetland O, Knudsen A, Dickstein K, Nilsen DW. Characteristics and prognostic impact of plasma fibrin monomer (soluble fibrin) in patients with coronary artery disease. Blood Coagul Fibrinolysis. 2002 ;13:301-8.
12. Koga S. A novel molecular marker for thrombus formation and life prognosis-clinical usefulness of measurement of soluble fibrin monomer-fibrinogen complex (SF) Rinsho Byori. 2004 ;52:355-61.
13. LaCapra S, Arkel YS, Ku DH, Gibson D, Lake C, Lam X. The use of thrombus precursor protein, D-dimer, prothrombin fragment 1.2, and thrombin antithrombin in the exclusion of proximal deep vein thrombosis and pulmonary embolism. Blood Coagul Fibrinolysis. 2000 ;11:371-7.
14. Mac Gillavry MR, Sanson BJ, de Monye W, Lijmer JG, Huisman MV, Buller HR, Nieuwenhuizen W, Brandjes DP. Use of a new monoclonal antibody-based enzyme immunoassay for soluble fibrin to exclude pulmonary embolism. ANTELOPE-Study Group. Thromb Haemost. 2000 ;84:474-7.
15. Nakahara K, Kazahaya Y, Shintani Y, Yamazumi K, Eguchi Y, Koga S, Wada H, Matsuda M. Measurement of soluble fibrin monomer-fibrinogen complex in plasmas derived from patients with various underlying clinical situations. Thromb Haemost. 2003 ;89:832-6.
16. Nieuwenhuizen W, Hoegee-De Nobel E, Laterveer R. A rapid monoclonal antibody-based enzyme immunoassay (EIA) for the quantitative determination of soluble fibrin in plasma. Thromb Haemost. 1992 ;68:273-7.
17. Nieuwenhuizen W. Soluble fibrin as a molecular marker for a pre-thrombotic state: a mini-review. Blood Coagul Fibrinolysis. 1993 ;4:93-6.
18. Okajima K, Uchiba M, Murakami K, Okabe H, Takatsuki K. Determination of plasma soluble fibrin using a new ELISA method in patients with disseminated intravascular coagulation. Am J Hematol. 1996 ;51:186-91:
19. Reber G, Bounameaux H, Perrier A, de Moerloose P. Performances of the fibrin monomer test for the exclusion of pulmonary embolism in symptomatic outpatients. Thromb Haemost. 1999 ;81:221-3.
20.Scarano L, Prandoni P, Gavasso S, Gomiero W, Carraro G, Girolami A.Blood Coagul Failure of soluble fibrin polymers in the diagnosis of clinically suspected deep venous thrombosis. Fibrinolysis. 1999 ;10:245-50.
21.Wada H, Sase T, Matsumoto T, Kushiya F, Sakakura M, Mori Y, Nishikawa M, Ohnishi K, Nakatani K, Gabazza EC, Shiku H, Nobori T. Increased soluble fibrin in plasma of patients with disseminated intravascular coagulation. Clin Appl Thromb Hemost. 2003 ;9:233-40.
22. Brummel KE, Butenas S, and Mann KG FXIII activation associated with a primary cleavage in the A-subunit at Arg-37 releasing an NH2-terminal activation peptide (54), is coincident with FPA release. J Biol Chem, 1999; 274:22862-22870
23. Nossel HL, Yudelman I, Canfield RE, Butler VP Jr, Spanondis K, Wilner GD, Qureshi GD. Measurement of fibrinopeptide A in human blood. J Clin Invest. 1974; 54:43-53
24.Shifman MA, Pizzo SV. The in vivo metabolism of antithrombin III and antithrombin III complexes. J Biol Chem. 1982 ;257 :3243-8.
25. Lee L. V., Ewald G. A., McKenzie C. R., ; Eisenberg P. R. The Relationship of SolubleFibrin and Cross-linked Fibrin Degradation Products to the Clinical Course of Myocardial Infarction. Arteriosclerosis, Thrombosis, and Vascular Biology. 1997, 17: 628-633.
26. Bounameaux H, Schneider PA, Slosman D, De Moerloose P, Reber G. Value of the plasma measurement of D-dimers in the diagnosis of venous thromboembolism J Mal Vasc. 1991;16:133-6.
27. Kelly J, Rudd A, Lewis RR, Hunt BJ. Plasma D-dimers in the diagnosis of venous thromboembolism. Arch Intern Med. 2002;162:747-56.
28. Kevorkian JP, Halimi C, Segrestaa JM, Drouet L, Soria C. Monitoring of patients with deep-vein thrombosis during and after anticoagulation with D-dimer. Lancet. 1998 ;351:571-2.
29. Kuruvilla J, Wells PS, Morrow B, MacKinnon K, Keeney M, Kovacs MJ. Prospective assessment of the natural history of positive D-dimer results in persons with acute venous thromboembolism (DVT or PE). Thromb Haemost. 2003;89:284-7.
30. Nunes JP. D-dimers in diagnosis of pulmonary embolism. Lancet. 2002 Aug 10;360(9331):489.
31. van Beek EJ, Schenk BE, Michel BC, van den Ende B, Brandjes DP, van der Heide YT, Bossuyt PM, Buller H R. The role of plasma D-dimers concentration in the exclusion of pulmonary embolism. Br J Haematol. 1996;92:725-32. Erratum in: Br J Haematol 1996; 95:218.
32.Perrier A, Roy PM, Aujesky D, Chagnon I, Howarth N, Gourdier AL, Leftheriotis G, Barghouth G, Comuz J, Hayoz D, Bounameaux H. Diagnosing pulmonary embolism in outpatients with clinical assessment, D-dimer measurement, venous ultrasound, and helical computed tomography: a multicenter management study. Am J Med. 2004 ;116:291-9.
33. Reber G, Bounameaux H, Perrier A, de Moerloose P. Evaluation of advanced D-dimer assay for the exclusion of venous thromboembolism. Thromb Res. 2002;107:197-200.
34.Wells PS, Anderson DR, Rodger M, Forgie M, Kearon C, Dreyer J, Kovacs G, Mitchell M, Lewandowski B, Kovacs MJ. Evaluation of D-dimer in the diagnosis of suspected deep-vein thrombosis N Engl J Med. 2003;349:1227-35.
35.Wells PS, Anderson DR, Rodger M, Stiell I, Dreyer JF, Bames D, Forgie M, Kovacs G, Ward J, Kovacs MJ. Excluding pulmonary embolism at the bedside without diagnostic imaging: management of patients with suspected pulmonary embolism presenting to the emergency department by using a simple clinical model and d-dimer. Ann Intem Med. 2001;135:98-107.

## Revendications

1. Méthode de diagnostic *in vitro* de l'activation de la coagulation liée à un risque de maladie thromboembolique, à partir d'un même échantillon sanguin prélevé chez un patient, comprenant :
i) la mesure de la quantité de produits de dégradation de la fibrine contenus dans l'échantillon testé, consistant en la mesure de la quantité de D-dimères présents dans l'échantillon et constituant le taux de D-dimères de base ;
ii) le traitement de l'échantillon par incubation avec un activateur de plasminogène de forte affinité pour la fibrine (Pa-Fb sp) dans des conditions permettant la dégradation de la fibrine soluble contenue dans l'échantillon, en produits de dégradation sans conduire à la dégradation du fibrinogène, et la mesure de la quantité de D-dimères contenus dans l'échantillon ainsi traité
iii) le calcul de la différence entre la quantité de D-dimères, mesurée après l'activation par l'activateur Pa-Fb sp à l'étape (ii) et la quantité de D-dimères avant ladite activation mesurée à l'étape (i), ladite différence constituant le taux de dégradation de la fibrine soluble (FSD) ;
iv) la comparaison du taux de D-dimères mesuré à l'étape (i) avec une valeur seuil normale déterminée de ce même produit de dégradation et la comparaison du taux de FSD calculé à l'étape (iii) avec une valeur seuil normale déterminée de FSD ;
le risque de maladie thromboembolique existant si l'un au moins du taux de D-dimères mesuré à l'étape (i) ou du taux de FSD calculés est supérieur à la valeur normale respective déterminée et l'exclusion dudit risque résultant lorsque le taux de D-dimères obtenu à l'étape (i) et le taux de FSD obtenu à l'étape (iii) sont inférieurs aux valeurs seuils normales respectives.

2. Méthode selon la revendication 1 pour la recherche d'une maladie veineuse thromboembolique.

3. Méthode selon la revendication 1 ou 2 pour l'application à la recherche de la formation d'un thrombus veineux.

4. Méthode selon l'une des revendications 1 à 3 pour le diagnostic d'une thrombose veineuse profonde.

5. Méthode selon l'une des revendications 1 à 3 pour le diagnostic d'une embolie pulmonaire.

6. Méthode de diagnostic selon l'une quelconque des revendications 1 à 5, dans laquelle l'échantillon sanguin est un échantillon de plasma.

7. Méthode selon l'une quelconque des revendications 1 à 6, dans laquelle l'échantillon sanguin est celui d'un patient bénéficiant d'une thérapie anti-coagulante.

8. Méthode selon l'une quelconque des revendications 5 à 7, dans laquelle l'activateur de plasminogène Pa-Fb sp est le tPA.

9. Méthode selon l'une quelconque des revendications 1 à 8, dans laquelle on prévient la dégradation du fibrinogène en ajoutant un inhibiteur de la plasmine.

10. Méthode selon la revendication 9, dans laquelle l'inhibiteur de la plasmine est l'aprotinine, qui est ajoutée après incubation de l'échantillon pendant 15 minutes à 37°C avec l'activateur de plasminogène Pa-Fb.

11. Méthode selon l'une quelconque des revendications 1 à 10, **caractérisée en ce que**, préalablement à l'incubation de l'échantillon avec l'activateur du plasminogène Pa-Fb, on ajoute à l'échantillon un anticoagulant tel qu'une composition d'acide citrique et de citrate trisodique.

12. Méthode selon l'une quelconque des revendications 1 à 11 comprenant en outre, le dosage des produits de dégradation de la fibrine soluble dans un échantillon contrôle négatif et dans un échantillon contrôle positif.

13. Méthode selon l'une quelconque des revendications 1 à 8, dans laquelle la mesure de D-dimères est réalisée au moyen d'anticorps monoclonaux anti-D-dimères.

14. Méthode de diagnostic selon l'une des revendications 1 à 9, comprenant en outre la mesure des FSD présents dans un échantillon témoin positif constitué par un échantillon de plasma normal au moyen d'étapes comprenant :
i. l'incubation d'un échantillon de plasma avec un activateur de la coagulation, de façon à permettre la formation de fibrine soluble sans la formation de caillot de fibrine, l'activateur étant par exemple une faible quantité de thrombine pour induire la formation de fibrine soluble,
ii. l'incubation de l'échantillon ainsi traité, avec un inhibiteur de la coagulation en quantité suffisante pour bloquer la coagulation, l'inhibiteur étant par exemple l'héparine ou l'hirudine,
iii. la mise en contact de l'échantillon préparé comprenant des monomères de fibrine soluble, avec un activateur de plasminogène de forte affinité pour la fibrine (Pa-Fb sp), notamment le t-PA, dans des conditions permettant la dégradation de la fibrine soluble contenue dans l'échantillon, en ses produits de dégradation, notamment en D-dimères, sans dégrader le fibrinogène.

15. Méthode de diagnostic selon l'une quelconque des revendications 1 à 10 dans laquelle la valeur seuil normale pour la quantité de D-dimères est de 500 ng/ml et la valeur seuil normale de FSD est de 300 ng/ml, le diagnostic d'exclusion de maladie thromboembolique étant prononcé lorsque les valeurs mesurées sur l'échantillon testé sont inférieures aux deux valeurs seuils respectives.

## Patentansprüche

1. In-vitro-Diagnoseverfahren zur Aktivierung der Koagulation in Zusammenhang mit einem thromboembolischen Erkrankungsrisiko ausgehend von einer bei einem Patienten genommenen gleichen Blutprobe, das umfasst:
i) Ermitteln der Menge der in der untersuchten Probe enthaltenen Fibrinabbauprodukte, wobei die Menge der in der Probe vorhandenen D-Dimere ermittelt wird, die den D-Dimer-Grundspiegel bildet;
ii) Behandeln der Probe durch Inkubation mit einem Plasminogenaktivator mit hoher Affinität zu Fibrin (Pa-Fb sp) unter Bedingungen, die das Zerlegen des in der Probe enthaltenen löslichen Fibrins in Abbauprodukte erlauben, ohne dass dies zum Abbau von Fibrinogen führt, und Ermitteln der Menge der in der so behandelten Probe enthaltenen D-Dimere;
iii) Berechnen des Unterschieds zwischen der in Schritt (ii) ermittelten D-Dimer-Menge nach Aktivierung durch den Aktivator Pa-Fb sp und der in Schritt (i) ermittelten D-Dimer-Menge vor dieser Aktivierung, wobei der Unterschied die Abbaumenge des löslichen Fibrins (SDF) darstellt;
iv) Vergleichen des in Schritt (i) ermittelten D-Dimer-Spiegels mit einem bestimmten normalen Grenzwert des gleichen Abbauprodukts und Vergleichen der in Schritt (iii) berechneten SDF-Menge mit einem bestimmten normalen SDF-Grenzwert; wobei das thromboembolische Erkrankungsrisiko besteht, wenn von dem in Schritt (i) ermittelten D-Dimer-Spiegel oder der berechneten SDF-Menge wenigstens ein Wert über dem bestimmten jeweiligen Normalwert liegt, und sich der Ausschluss dieses Risikos ergibt, wenn der in Schritt (i) erhaltene D-Dimer-Spiegel und die in Schritt (iii) erhaltene SDF-Menge unter den jeweiligen normalen Grenzwerten liegen.

2. Verfahren nach Anspruch 1,
zur Untersuchung einer thromboembolischen venösen Erkrankung.

3. Verfahren nach Anspruch 1 oder 2,
zur Anwendung auf die Untersuchung der Bildung eines venösen Thrombus.

4. Verfahren nach einem der Ansprüche 1 bis 3,
zur Diagnose einer tiefen Venenthrombose.

5. Verfahren nach einem der Ansprüche 1 bis 3,
zur Diagnose einer Pulmonalembolie.

6. Verfahren nach einem der Ansprüche 1 bis 5,
bei dem die Blutprobe eine Plasmaprobe ist.

7. Verfahren nach einem der Ansprüche 1 bis 6,
bei dem die Blutprobe von einem Patienten stammt, der mit einer Antikoagulationstherapie behandelt wird.

8. Verfahren nach einem der Ansprüche 5 bis 7,
bei dem tPA der Plasminogenaktivator Pa-Fb sp ist.

9. Verfahren nach einem der Ansprüche 1 bis 8,
bei dem der Fibrinogenabbau durch Hinzufügen eines Plasmininhibitors verhindert wird.

10. Verfahren nach Anspruch 9,
bei dem der Plasmininhibitor Aprotinin ist, das nach Inkubation der Probe mit dem Plasminogenaktivator Pa-Fb für die Dauer von 15 Minuten bei 37 °C hinzugefügt wird.

11. Verfahren nach einem der Ansprüche 1 bis 10,
**dadurch gekennzeichnet, dass** der Probe vor der Inkubation der Probe mit dem Plamsinogenaktivator Pa-Fb ein Antikoagulans hinzugefügt wird, zum Beispiel eine Zusammensetzung aus Zitronensäure und Trinatriumcitrat.

12. Verfahren nach einem der Ansprüche 1 bis 11,
das ferner die Bestimmung der Abbauprodukte des löslichen Fibrins in einer negativen Kontrollprobe und in einer positiven Kontrollprobe umfasst.

13. Verfahren nach einem der Ansprüche 1 bis 8,
bei dem das Ermitteln der Di-Dimere mit Hilfe von monoklonalen Anti-D-Dimer-Antikörpern erfolgt.

14. Verfahren nach einem der Ansprüche 1 bis 9,
das ferner das Ermitteln der SDF umfasst, die in einer aus einer normalen Plasmaprobe bestehenden positiven Kontrollprobe vorhanden sind, mit Hilfe von Schritten,
die umfassen:
i. Inkubieren einer Plasmaprobe mit einem Koagulationsaktivator, um die Bildung von löslichem Fibrin ohne die Bildung von Fibringerinnseln zu ermöglichen, wobei der Aktivator zum Beispiel eine geringe Menge Thrombin ist, um die Bildung von löslichem Fibrin zu bewirken;
ii. Inkubieren der so behandelten Probe mit einem Koagulationsinhibitor in ausreichender Menge zum Hemmen der Koagulation, wobei der Inhibitor zum Beispiel Heparin oder Hirudin ist;
iii. Inkontaktbringen der lösliche Fibrinmonomere enthaltenden hergestellten Probe mit einem Plasminogenaktivator mit hoher Affinität zu Fibrin (Pa-Fb sp), insbesondere t-PA, unter Bedingungen, die das Zerlegen des in der Probe enthaltenen löslichen Fibrins in seine Abbauprodukte, insbesondere in D-Dimere, erlauben, ohne hierbei Fibrinogen abzubauen.

15. Verfahren nach einem der Ansprüche 1 bis 10,
bei dem der für D-Dimere normale Grenzwert 500 ng/ml und der normale SDF-Grenzwert 300 ng/ml beträgt, wobei die Diagnose zum Ausschluss von thromboembolischer Erkrankungen angezeigt ist, wenn die bei der untersuchten Probe gemessenen Werte kleiner sind als die beiden jeweiligen Grenzwerte.

## Claims

1. An *in vitro* diagnostic method for diagnosing coagulation activation linked to a risk of thromboembolic disease starting from a blood sample removed from a patient, comprising:
i) measuring the quantity of fibrin degradation products contained in the test sample, consisting of measuring the quantity of D-dimers present in the sample and constituting the base level of D-dimers;
ii) treating the sample by incubation with a plasminogen activator with a high affinity for fibrin (Pa-Fb sp) under conditions allowing degradation of the soluble fibrin contained in the sample into degradation products without resulting in the degradation of fibrinogen, and measuring the quantity of D-dimers contained in the treated sample;
iii) calculating the difference between the quantity of D-dimers measured after activation by the Pa-Fb sp activator in step (ii) and the quantity of D-dimers before said activation measured in step (i), said difference constituting the degree of degradation of the soluble fibrin (SDF);
iv) comparing the level of D-dimers measured in step (i) with a normal threshold value determined for said degradation product and comparing the level of SDF calculated in step (iii) with a normal threshold value determined for SDF;
a risk of thromboembolic disease existing if at least one of the levels of D-dimers measured in step (i) or the calculated level of SDF is higher than the respective determined normal value and said risk being excluded when the level of D-dimers obtained in step (i) and the level of SDF obtained in step (iii) are lower than the respective normal threshold values.

2. A method according to claim 1, for investigating a thromboembolic venous disease.

3. A method according to claim 1 or 2, applied to investigating the formation of a venous thrombus.

4. A method according to any one of claims 1 to 3, for the diagnosis of a deep venous thrombosis.

5. A method according to any one of claims 1 to 3, for the diagnosis of a pulmonary embolism.

6. A diagnostic method according to any one of claims 1 to 5, in which the blood sample is a plasma sample.

7. A method according to any one of claims 1 to 6, in which the blood sample is that of a patient benefiting from an anti-coagulation therapy.

8. A method according to any one of claims 5 to 7, in which the Pa-Fb sp plasminogen activator is t-PA.

9. A method according to any one of claims 1 to 8, in which fibrinogen degradation is prevented by adding a plasmin inhibitor.

10. A method according to claim 9, in which the plasmin inhibitor is aprotinin, which is added after incubating the sample for 15 minutes at 37°C with the Pa-Fb plasminogen activator.

11. A method according to any one of claims 1 to 10, **characterized in that** prior to incubation of the sample with the Pa-Fb plasminogen activator, an anti-coagulant such as a composition of citric acid and trisodium citrate is added to the sample.

12. A method according to any one of claims 1 to 11, further comprising assaying the degradation products of soluble fibrin in a negative control sample and in a positive control sample.

13. A method according to any one of claims 1 to 8, in which the D-dimers are measured using anti-D-dimer monoclonal antibody.

14. A diagnostic method according to any one of claims 1 to 9, further comprising measuring the SDF present in a positive control sample constituted by a normal plasma sample by carrying out steps comprising:
i) incubating a plasma sample with a coagulation activator in order to allow the formation of soluble fibrin without forming a fibrin clot, the activator being, for example, a small quantity of thrombin to induce the formation of soluble fibrin;
ii) incubating the treated sample with a coagulation inhibitor in a quantity sufficient to block coagulation, the inhibitor being heparin or hirudin,
iii) bringing the prepared sample comprising soluble fibrin monomers into contact with a plasminogen activator with a high affinity for fibrin (Pa-Fb sp), especially t-PA, under conditions allowing degradation of the soluble fibrin contained in the sample into its degradation products, especially D-dimers, without degrading the fibrinogen.

15. A diagnostic method according to any one of claims 1 to 10, in which the normal threshold value for the quantity of D-dimers is 500 ng/ml and the normal threshold value for SDF is 300 ng/ml, a diagnosis excluding thromboembolic disease being made when the measured values of the test sample are less than the two respective threshold values.
